(19) Europäisches Patentamt
European Patent Office
Office européen des brevets

(11) Veröffentlichungsnummer: **0 525 814 A1**

(12) # EUROPÄISCHE PATENTANMELDUNG

(21) Anmeldenummer: **92113214.8**

(22) Anmeldetag: **03.08.92**

(51) Int. Cl.$^5$: **A61K 31/165**, A61K 31/17, A61K 31/495

(30) Priorität: **02.08.91 DE 4125671**
**14.02.92 DE 4204405**

(43) Veröffentlichungstag der Anmeldung: **03.02.93 Patentblatt 93/05**

(84) Benannte Vertragsstaaten: **PT**

(71) Anmelder: **BOEHRINGER MANNHEIM GMBH**
**Sandhofer Strasse 116**
**W-6800 Mannheim 31(DE)**

(72) Erfinder: **Dreckmann-Behrendt, Bruno, Dr.**
**Dürerstrasse 53**
**W-6800 Mannheim 1(DE)**
Erfinder: **Heck, Reinhard, Dr.**
**Ulmenweg 12**
**W-6718 Grünstadt 3(DE)**
Erfinder: **Wolff, Hans-Peter, Dr.**
**Untere Clignetstrasse 4**
**W-6800 Mannheim 1(DE)**
Erfinder: **Dresel, Alois, Dr.**
**Mozartstrasse 1**
**W-6905 Schriesheim(DE)**
Erfinder: **Wiest, Edith, Dr.**
**Eckbachring 9**
**W-6711 Heuchelheim(DE)**

(54) **Verwendung von Di-tert.-Butylhydroxyphenyl-Derivaten zur Therapie von entzündlichen Erkrankungen des Magen-Darm-Traktes.**

(57) Verwendung von BHT-Verbindungen der Formel I

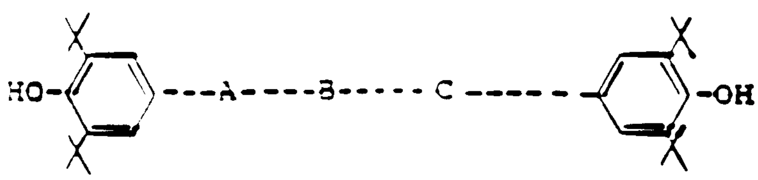

(I)

in der die Gruppe A-B-C die in den Patentansprüchen angegebene Bedeutung hat, zur Behandlung von entzündlichen Erkrankungen des Magen-Darm-Traktes.

EP 0 525 814 A1

Die vorliegende Erfindung betrifft die Verwendung von Di-tert.-Butylhydroxyphenyl-Derivaten zur Herstellung von Arzneimitteln zur Therapie von entzündlichen Erkrankungen des Magen-Darm-Traktes.

Infiltration von Geweben durch Zellen des Immunsytems, deren Produkte Gewebezerstörungen induzieren, wie polymorphkernigen Leukozyten, T- und B-Lymphocyten, Monocyten, Makrophagen, Blutplättchen und anderen mehr, führt zu entzündlichen Gewebereaktionen. Wichtige Produkte hierbei sind reaktive Sauerstoff-Metabolite sowie eine Vielzahl von Mediatoren, beispielweise Leukotriene, Prostaglandine oder Thromboxan. Sauerstoff-Metabolite werden von den phagocytierenden Zellen freigesetzt. Sie führen neben direkter Gewebeschädigung zur Aktivierung von Oxidasen, wie Lipoxygenasen, Phospholipase $A_2$, NADPH-Oxidase oder Myeloperoxidase. Lipoxygenasen induzieren ihrerseits die Synthese von Leukotrienen, die chemotaktisch für polymorphkernige Leukozyten und Makrophagen wirken und so weitere Entzündungszellen anlocken. Dies führt zur Amplifiktion der einmal angestoßenen Reaktion und endet mit den beobachteten Gewebezerstörungen.

Die Pathogenese von entzündlichen Erkrankungen des Magen-Darm-Traktes ist bis zum heutigen Tag nicht eindeutig geklärt. Ein oder mehrere unbekannte auslösende Faktoren führen zu einer Entzündungsantwort in der Mukosa des Magen-Darm-Traktes. Diskutiert werden neben Ischämie/Reperfusions Phänomenen auch Autoimmunreaktionen, Infektionen und im Duodenum direkte Schädigungen durch erhöhte Magensäureproduktion. Die infiltrierten Entzündungszellen produzieren lösliche Mediatoren, die eine Amplifikation der Entzündungsantwort auslösen. Während die Prostaglandine heute eher als sekundär eingestuft werden, haben Leukotriene, insbesondere Leukotrien $B_4$ ($LTB_4$), aufgrund ihrer potenten chemotaktischen und chemokinetischen Fähigkeiten, proinflammatorische Eigenschaften.

Die Rekrutierung und Akkumulation weiterer Entzündungszellen am Entzündungsort ist ein wichtiger Faktor der Entzündungsverstärkung, vermittelt durch $LTB_4$ (Wallace, TIPS 11:51-52, 1991).

Mukosaschädigungen wie Erosionen, Ulcera und Blutungen mit Verlust spezifischer Funktionenen, wie Rückresorption von Wasser und Elektrolyten, sind die pathologisch-histologischen bzw. funktionell faßbaren Manifestationen dieses Stadiums. Letztlich können diese Prozesse zu chronischen Ulcera und Funktionsverlust der Mukosa in einem solchen Ausmaß führen, daß Morbidität eintritt. Wirkstoffe, die diese Prozesse verhindern oder stark einschränken, sind bei einer Reihe von pathologischen Zuständen von therapeutischem Wert.

Der vorliegenden Erfindung liegt demnach die Aufgabe zugrunde, geeignete Arzneimittel zur Verfügung zu stellen, die zur Behandlung von entzündlichen Erkrankungen des Magen-Darm-Traktes verwendet werden können.

Es wurde nun überraschenderweise gefunden, daß Di-tert.-Butylhydroxyphenyl-Derivate der unten genannten allgemeinen Formel I das Ausmaß von entzündlichen Reaktionen der Darmmukosa herabsetzen.

Di-tert.-Butylhydroryphenyl-Derivate der allgemeinen Formel I vermögen in der Ratte das Ausmaß einer Säure-induzierten Kolitis zu reduzieren. Dieser Effekt ist auf ihre antioxidative und Lipoxygenase-hemmende Wirkung zurückzuführen. Sie gelangen aufgrund ihrer schlechten Resorbierbarkeit direkt an den Ort der Entzündung in der Mukosa und können so lokal hohe Konzentrationen bilden. Dieser Effekt ist unabhängig von ihrer Verwendung als Arzneimittel zur Therapie von Arteriosklerose, wie bereits beansprucht.

Die Substanzen eignen sich daher zur Behandlung akut- und chronisch-entzündlicher Darmerkrankungen unterschiedlicher Ätiologie wie Proktitis, Ileitis, Kolitis ulcerosa und Morbus Crohn sowie Ulcus duodeni und Uclus ventriculi, ohne den Nachteil einer systemischer Nebenwirkungen.

Für die vorliegende Erfindung geeignete Di-tert.-Butylhydroxyphenyl-Derivate (BHT-Derivate) sind BHT-Derivate der allgemeinen Formel I

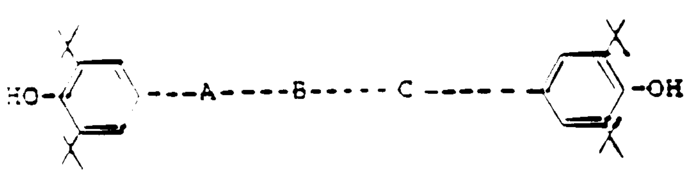

(I)

in der

A und C, die gleich oder verschieden sein können, eine Bindung oder eine gesättigte oder ungesättigte Kohlenwasserstoffkette mit 1-8 C-Atomen, die verzweigt oder unverzweigt sowie durch Heteroatome wie z.B. Sauerstoff oder Schwefel unterbrochen sein kann

und B eine der folgenden Gruppen darstellt:

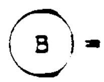

a) 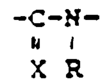

b) 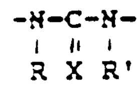

c) 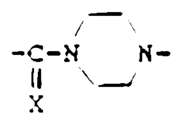

d) 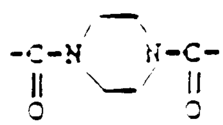

e) 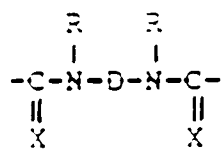

f) 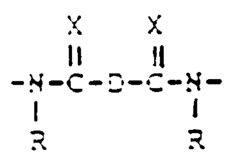

in denen

X Sauerstoff oder Schwefel,

R,R', welche gleich oder verschieden sein können und Wasserstoff oder $C_1$-$C_4$ Alkyl, bevorzugt Methyl oder Ethyl, bedeuten,

und

D für eine ungesättigte oder gesättigte, verzweigte oder unverzweigte Kette mit ein bis 10 C-Atomen, welche durch ein oder mehrere Heteroatome - bevorzugt durch Schwefel - unterbrochen sein kann, steht.

Bevorzugte Bedeutung von A bzw. C sind Valenz, $-CH_2-CH_2-$,

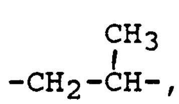, 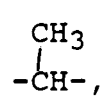, 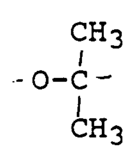

oder -CH = CH-.

D stellt bevorzugt eine $-CH_2-$, $-CH_2-CH_2-$, $(CH_2)_4-$, $(CH_2)_6$, $-CH_2-CH_2-O-CH_2-CH_2$ oder $-CH_2-CH_2-S-$

$CH_2$-$CH_2$-Gruppe dar.

Die Verbindungen der Formel I sowie deren Herstellung sind in EP-A-404 039 beschrieben. Es wird in dieser Patentanmeldung darauf hingewiesen, daß die Verbindungen aufgrund ihrer anti-oxidierenden Eigenschaften, stabilisierende Wirkung auf Lipoproteine und Hemmwirkung auf die Reacylierung des LDL-Cholesterins in Makrophagen Verwendung als Arzneimittel finden, insbesondere als Anti-arteriosklerotica.

Zur Herstellung von Arzneimitteln für die Therapie von entzündlichen Reaktionen des Magen-Darm-Traktes werden Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der Allgemeinen Formel I können in flüssiger oder fester Form oral, rektal und parenteral appliziert werden. Als Lösungsmittel kommt Öl aber auch Wasser, das Stabilisierungsmittel, Lösungsvermittler und/oder Puffer enthält in Frage. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Vikositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylengykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 1-100 mg/kg Körpergewicht.

Gegenstand der Erfindung sind Verbindungen der Formel I, insbesondere die folgenden Verbindungen:

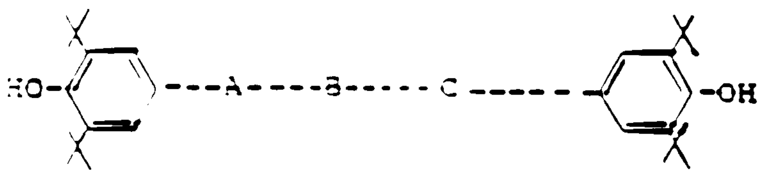

Das Symbol "X" in der Formel I bedeutet "tert. Butyl".

| Bsp. | - A - B - - C - | Fp.° C |
|---|---|---|
| 1 | $-CH_2-CH_2-CO-NH-CH_2-CH_2-$ | 172 |
| 2 | $-CH_2CH_2-NH-CS-NH-CH_2CH_2-$ | 135-136 |
| 3 | $-CS-NH-$ | 165-166 |
| 4 | $-CH=CH-CS-NH-CH_2-CH_2-$ | 244 |
| 5 | $-CS-NH-CH_2-CH_2-$ | 202-203 |
| 6 | $-CH_2-CH_2-CS-NH-CH_2CH_2-$ | 185 |
| 7 | $-CH_2CH_2-CONH(CH_2)_6NHCO-CH_2CH_2-$ | 156 |
| 8 | $-CH_2CH_2-CO-N(CH_3)-CH_2CH_2-$ | 116-118 |
| 9 | $-CH_2-CO-NH-CH_2-CH_2-$ | 163 |
| 10 | $-CH_2-CH_2-NH-CO(CH_2)_4CO-NH-CH_2-CH_2-$ | 187 |
| 11 | $-CH_2-CH_2-N-CO(CH_2)_4CO-N-CH_2-CH_2-$<br>\| \|<br>Et Et | 175 |
| 12 | $-CH_2-CH_2-N-CO-CH_2-CH_2-$<br>\|<br>Et | 126-128 |

Die pharmakologischen Untersuchungen, die für sämtliche genannten Verbindungen der allgemeinen Formel I als repräsentativ angesehen werden können, wurden mit der Verbindung des Beispieles 1 aus Tabelle 1 durchgeführt. Die Verbindung ist in EP-A-404 039 aufgeführt. Als Vergleichssubstanz wurde Probucol$^R$ mitgeführt.

## Therapeutische Wirkung

Die therapeutische Wirkung von Beispiel 1 bei Entzündungen des Magen-Darm-Traktes kann dem nachfolgenden Versuch entnommen werden. Der Versuch ist so beschrieben, daß jedermann, der die nötigen Kenntnisse und Einrichtungen besitzt, ihn nachvollziehen kann. Es ist nicht beabsichtigt durch die Auswahl der durchgeführten Experimente in irgendwelcher Weise den Bereich der Anwendung der Erfindung zu beschränken.

### a) Allgemeines Prinzip

Die Verabreichung von Essigsäure über das Rektum an Ratten führt zu Mukosa- und Submukosa-Veränderungen wie sie beim Kolitis-Patienten gefunden werden (Mac Pherson und Pfeiffer; Digestion 17:135-150, 1978). Es treten Gewebeschwellungen, Einblutungen sowie massive Darmulcera auf. Beispiel 1 und Probucol wurden in einer Dosierung von 200 mg/kg/d 3 mal oral verabreicht. Die Behandlung wurde einen Tag vor Entzündungsinduktion mit 5% Essigsäure begonnen. Die Studie gibt Aufschluß ob Beispiel 1 eine Wirkung auf entzündliche Prozesse im Darm besitzt. Das Ausmaß einer entzündlichen Reaktion im Darm wird durch Begutachtung des Mukosa-Zustandes und Klassifizierung nach festgelegten Parametern bestimmt.

### b) Versuchsbeschreibung

Akute Kolitis wurde bei 24 h nüchternen Ratten durch rektale Instillation von 5% Essigsäure (1,5ml/Tier) über eine 8 cm lange Sonde induziert. Die morphologische Auswertung des Mukosazustandes aus dem Rektum und Teilen des distalen Kolons (ca. 8 cm) erfolgte am 3. Tag nach Entzündungsinduktion, nachdem

die Tiere getötet worden waren. Beispiel 1 und Probucol wurden in einer Dosierung von 200 mg/kg/d 3 mal p.o. (2,5 ml/kg) verabreicht. Die erste Gabe erfolgte 24 h vor Essigsäure-Applikation. Kontrolltieren wurde das Substanzlösungsmittel (20% DMSO in Öl) verabreicht.

Die morphologische Auswertung erfolgte an einem computergeschützten Bildanalyse-System. Es wurde der Anteil von entzündetem Gewebe an der Gesamtdarmoberfläche bestimmt. Folgende Schädigungsstufen wurden unterschieden:

- Stufe 0 : ohne Befund
- Stufe 1 : Schwellung des Gewebes mit leichter Rötung
- Stufe 2 : starke Rötung des Gewebes mit Einblutungen oder ulcerierenden Nekrosen

**c) Ergebnisse**

Die Ergebnisse der morphologischen Auswertung von Darmabschnitten aus Kontrolltieren und Tieren unter Beispiel 1 sind in Tab. 2 und Abb. 1 dargestellt.

Drei Tage nach rektaler Applikation von 5%iger Essigsäure an Ratten wiesen große Bereiche der inneren Darmoberfläche aus dem Bereich des distalen Kolons und Rektums Einblutungen und ulcerierende Nekrosen auf (Kontrollgruppe aus Tab. 2; Schädigungsstufe 2). Die übrigen Gewebebereiche waren zumindest leicht gerötet und geschwollen (Schädigungsstufe 1).

Mehrmalige Behandlung mit Probucol (3 x 200 mg/kg p.o.) hatte keinen Einfluß auf die durch Essigsäure-Applikation hervorgerufenen Mukosaschädigungen (Tab. 2).
Im Gegensatz dazu, führte die Behandlung mit Beispiel 1 (3 x 200 mg/kg p.o.; Tab. 2) zu deutlicher Abnahme von stark geschädigten Darmanteilen (Schädigungsstufe 2) und gleichzeitiger Zunahme von nur noch geschwollenen Gewebebereichen (Schädigungsstufe 1). Es waren sogar wieder größere, morphologisch normale Gewebebereiche (Schädigungsstufe 0) nachweisbar. Die beobachteten Unterschiede waren für alle 3 Schädigungsstufen signifikant ($p<0,01$; Student's t-Test). Hiermit ist anhand von Beispiel 1 eindeutig gezeigt, daß Verbindungen der allgemeinen Formel I das Ausmaß von Entzündungen des Gastrointestinaltraktes reduzieren.

Tab. 2 Anteil von Mukosabereichen (in %) unterschiedlicher Schädigungsstufe an der Gesamtfläche des untersuchten Darmbereichs aus Rektum und Teilen des distalen Kolons 3 Tage nach Entzündunginduktion: Vergleich von Behandlung mit Beispiel 1 (3 x 200 mg/kg p.o.), Probucol (3 x 200 mg/kg p.o.) und Lösungsmittel (20 % DMSO in Öl; Kontrolle)

| Schädigungs-stufe § | Kontrolle (n=15) | Beispiel 1 (n=10) | Probucol (n=5) |
|---|---|---|---|
| 0 | 0,60 ± 0,60 | 17,20 ± 8,93* | 0,12 ± 0,09 |
| 1 | 41,96 ± 4,61 | 66,96 ± 8,31* | 52,78 ± 9,36 |
| 2 | 57,38 ± 4,75 | 15,75 ± 6,24* | 47,19 ± 9,36 |

§ Schädigungsstufe 0 : ohne Befund
Schädigungsstufe 1 : Schwellung des Gewebes mit leichter Rötung
Schädigungsstufe 2 : starke Rötung des Gewebes mit Einblutungen oder ulcerierenden Nekrosen

* Signifikanter Unterschied im Vergleich zur Kontrolle (p<0,01)

## Patentansprüche

1. Verwendung von Verbindungen der allgemeinen Formel I

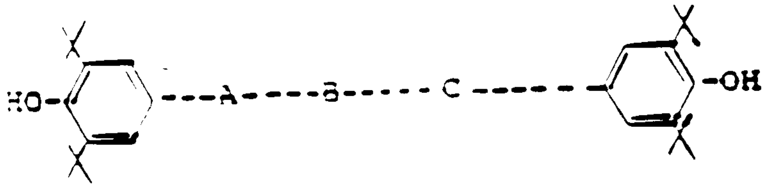

(I)

in der

A und C, die gleich oder verschieden sein können, eine Bindung oder eine gesättigte oder ungesättigte Kohlenwasserstoffkette mit 1-8 C-Atomen, die verzweigt oder unverzweigt sowie durch Heteroatome wie z.B. Sauerstoff oder Schwefel unterbrochen sein kann

und B eine der folgenden Gruppen darstellt:

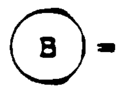

a) 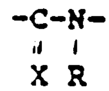

b) 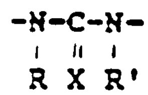

c) 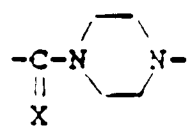

d) 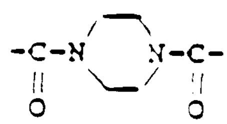

e) 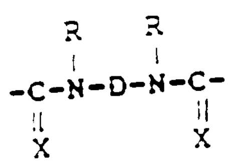

f) 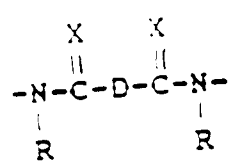

in denen

X Sauerstoff oder Schwefel,

R,R', welche gleich oder verschieden sein können und Wasserstoff oder $C_1$-$C_4$ Alkyl, bevorzugt Methyl oder Ethyl, bedeuten,

und

D für eine ungesättigte oder gesättigte, verzweigte oder unverzweigte Kette mit ein bis 10 C-Atomen, welche durch ein oder mehrere Heteroatome - bevorzugt durch Schwefel - unterbrochen sein kann, steht.

zur Behandlung von entzündlichen Erkrankungen des Magen-Darm-Traktes.

**2.** Verwendung von Verbindungen der Formel I gemäß Anspruch 1, in denen die Gruppe A-B-C

a) $-CH_2-CH_2-CO-NH-CH_2-CH_2-$
b) $-CH_2-CH_2-NH-CS-NH-CH_2-CH_2-$
c) $-CS-NH-$
d) $-CH=CH-CS-NH-CH_2-CH_2-$
e) $-CS-NH-CH_2-CH_2-$
f) $-CH_2-CH_2-CS-NH-CH_2-CH_2-$
g) $-CH_2-CH_2-CO-NH-(CH_2)_6-NH-CO-CH_2-CH_2-$
h) $-CH_2-CH_2-CO-N(CH_3)-CH_2-CH_2-$
i) $-CH_2-CO-NH-CH_2-CH_2-$
j) $-CH_2-CH_2-NH-CO(CH_2)_4\ CO-NH-CH_2-CH_2-$

k) 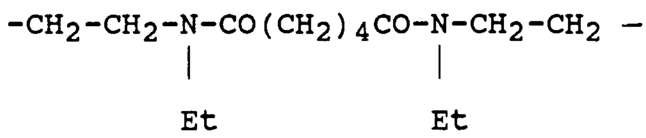

l) 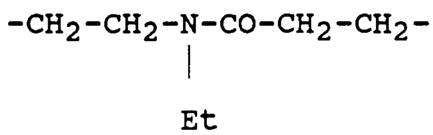

darstellt.

**3.** Verwendung von Verbindungen gemäß Anspruch 1 oder 2 zur Behandlung der Kolitis.

Europäisches Patentamt

# EUROPÄISCHER RECHERCHENBERICHT

Nummer der Anmeldung

EP 92 11 3214

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,P | EP-A-0 474 403 (ELI LILLY)<br>* Anspruch 1; Seite 8, Zeilen 50-57; Seite 7, Zeilen 30-32 *<br>--- | 1-3 | A 61 K 31/165<br>A 61 K 31/17<br>A 61 K 31/495 |
| X,Y<br>D | EP-A-0 404 039 (BOEHRINGER MANNHEIM)<br>* Seite 3, Zeilen 45-51; Ansprüche *<br>--- | 1-3 | |
| Y | AGENTS AND ACTIONS, Band 12, Nr. 5, 1982, Seiten 674-683, Birkhäuser Verlag, Basel DE; G.G.I. MOORE et al.: "2,6-Di-tert-butyl-4-(2'-thenoyl)phenol(R-830): a novel nonsteroidal anti-inflammatory agent with antioxidant properties"<br>* Seiten 681-682 *<br>--- | 1-3 | |
| Y | EP-A-0 293 899 (SEARLE & CO.)<br>* Seite 2, Zeilen 1-5; Anspruch 1 *<br>----- | 1-3 | |
| | | | **RECHERCHIERTE SACHGEBIETE (Int. Cl.5)** |
| | | | A 61 K |

Der vorliegende Recherchenbericht wurde für alle Patentansprüche erstellt

| Recherchenort | Abschlußdatum der Recherche | Prüfer |
|---|---|---|
| DEN HAAG | 18-09-1992 | GERLI P.F.M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)